(19) Europäisches Patentamt
European Patent Office
Office européen des brevets

(11) Publication number: **0 138 303**
**A1**

# (12) EUROPEAN PATENT APPLICATION

(21) Application number: **84304797.8**

(22) Date of filing: **13.07.84**

(51) Int. Cl.⁴: **C 07 C 143/66,** C 09 B 29/30

(30) Priority: **19.08.83 GB 8322398**

(43) Date of publication of application: **24.04.85**
**Bulletin 85/17**

(84) Designated Contracting States: **AT CH DE FR GB IT LI**

(71) Applicant: **IMPERIAL CHEMICAL INDUSTRIES PLC, Imperial Chemical House Millbank, London SW1P 3JF (GB)**

(72) Inventor: **Hughes, Nigel, 7 Leonardin Close, Shaw Oldham OL2 7NH (GB)**

(74) Representative: **Stephenson, Kenneth et al, Imperial Chemical Industries PLC Legal Department: Patents PO Box 6, Welwyn Garden City Herts, AL7 1HD (GB)**

## (54) Naphthalene sulphonic acids.

(57) Naphthol sulphonic acids of the formula:

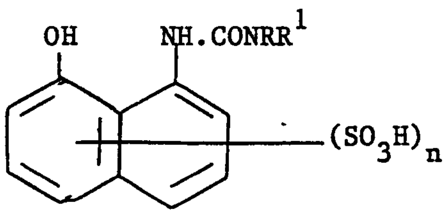

(I)

where

n is 1 or 2;

R is H or an optionally substituted alkyl;

$R^1$ is H, optionally substituted alkyl or optionally substituted cycloalkyl,

or R and $R^1$ together with the nitrogen atom form a heterocyclic ring.

The use of the naphthol sulphonic acids as coupling components in the preparation of azo dyes, especially acid, direct and reactive dyes, is described.

EP 0 138 303 A1

NAPHTHALENE SULPHONIC ACIDS

This invention relates to naphthalene sulphonic acids especially ureido naphthol sulphonic acids which are useful as coupling components in the manufacture of azo dyestuffs.

It is well known to use aminonaphthol sulphonic acids as coupling components in the manufacture of azo dyestuffs. Also it is well known to use as coupling components derivatives of these compounds in which the amino group is converted to an amido group e.g. an acetylamino or benzoylamino group.

We have now found a novel class of naphthol sulphonic acids which when used as coupling components lead to azo dyes, especially reactive dyes, with desirable properties such as high solubility combined with strong colouring power for textile substrates.

According to the present invention there are provided naphthol sulphonic acids of the formula:

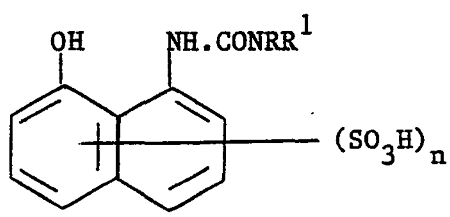 (1)

where n is 1 or 2;

R is H or an optionally substituted alkyl;

$R^1$ is H, optionally substituted alkyl or optionally substituted cycloalkyl,

or R and $R^1$ together with the nitrogen atom form a heterocyclic ring.

Preferred naphthol sulphonic acids have n as 2 and especially are of the formula:

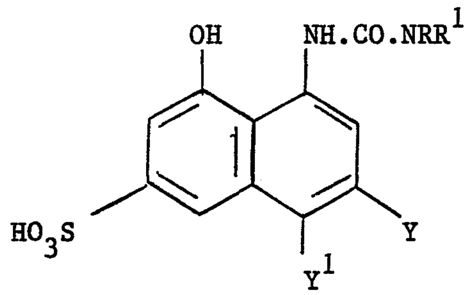

(2)

where one of $Y^1$ and Y is $SO_3H$ and the other H.

Especially preferred naphthol sulphonic acids are of the formula:

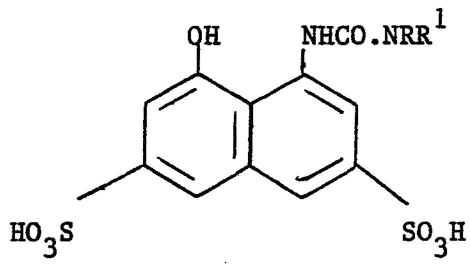

(3)

As examples of alkyl groups represented by R and $R^1$ there may be mentioned $C_{1-8}$ especially $C_{1-4}$ alkyl groups such as methyl, ethyl, propyl, butyl, hexyl and octyl and these groups with substituents (particularly two and especially one) such as OH, CN, $OSO_3H$, $SO_3H$, amino, mono or di-$C_{1-4}$ alkylamino, aminoalkylamino, $C_{1-4}$ alkoxy or hydroxy $C_{1-4}$ alkoxy, for example hydroxyethyl, hydroxypropyl, cyanoethyl, sulphatoethyl, sulphoethyl, aminobutyl, N-methylaminopropyl, N,N-dimethylaminopropyl, N-aminoethylaminoethyl, methoxyethyl, hydroxyethoxyethyl and hydroxypropoxypropyl.

As examples of cycloalkyl groups represented by $R^1$ there may be mentioned cyclohexyl and methylcyclohexyl.

When R and $R^1$ together with the nitrogen atom form a heterocyclic ring this ring may optionally contain further hetero atoms in addition to the nitrogen shown in formula (1). Examples of such heterocyclic rings include

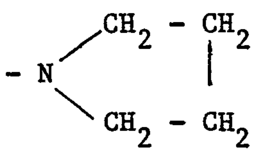

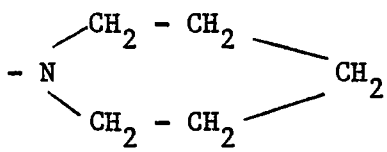

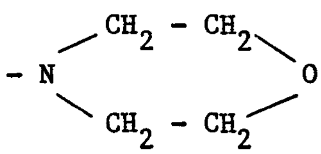

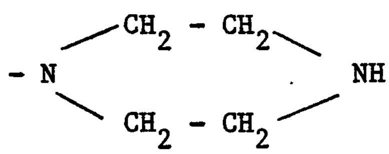

The preferred naphthol sulphonic acids are those of formula (3) in which R and $R^1$ are each independently selected from H and $C_{1-4}$ alkyl, optionally substituted with OH, CN, mono or di $C_{1-4}$ alkylamino or hydroxy $C_{1-4}$ alkoxy or R and $R^1$ together are $(CH_2)_{4 \text{ or } 5}$, $(CH_2)_2O(CH_2)_2$, $(CH_2)_2NH(CH_2)_2$ or $R^1$ is cyclohexyl.

It will be appreciated that the naphthol sulphonic acids shown herein in the free acid form may be readily converted to salts e.g. ammonium, sodium or potassium and will be conveniently handled in such a form especially in aqueous solutions.

The naphthol sulphonic acids of formula (1) may be manufactured by a number of processes depending to some extent on the nature of R and $R^1$.

The naphthol sulphonic acids of formula (1) in which R and $R^1$ are both H may be obtained by reacting aminonaphthol sulphonic acids of the formula:

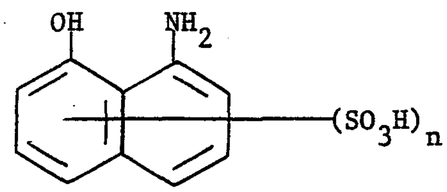

(4)

where n is 1 or 2 with metal cyanates e.g. sodium cyanate under acid conditions.

The reaction is conducted in aqueous media at 0-25°C. The product is not usually isolated but used in solution for coupling reactions. On heating, the naphthol sulphonic acids of formula (1) with $R = R^1 = H$ convert to cyclic urethanes of the formula:

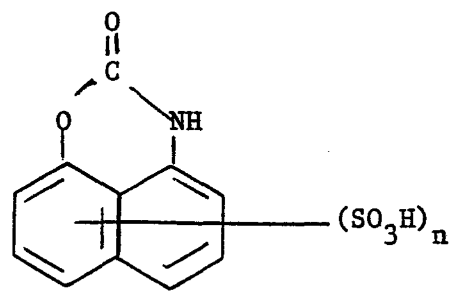

(5)

which may be alternatively obtained by reacting the aminonaphthol sulphonic acid of formula (4) with phosgene.

As examples of aminonaphthol sulphonic acids of formula (4) there may be mentioned S-, K- and especially H-acids.

A wide variety of naphthol sulphonic acids of formula (1) may be obtained by reacting the cyclic urethanes of formula (5) with an amine of the formula $H.NRR^1$.

This reaction may be carried out in alcoholic media especially methanolic media at ambient temperature or with slight heating e.g. at temperatures of 40-80°C. The reaction may also be conducted in aqueous media but this often leads to a minor proportion of unwanted products e.g. aminonaphthol sulphonic acid of formula (4). The reaction mixture is usually used for further reaction e.g. couplings without purification though product may be isolated if desired by conventional means.

As examples of amines of the formula $H.NRR^1$ there may be mentioned ammonia, methylamine, ethylamine, diethylamine, butylamine, methylhexylamine, beta-cyanoethylamine, ethanolamine, diethanolamine, taurine, N,N-dimethylaminopropylamine, N-methylethanolamine, cyclohexylamine, morpholine, piperidine, piperazine and pyrrolidine.

Some naphthol sulphonic acids of formula (1) in which $R^1$ is optionally substituted alkyl or optionally substituted cycloalkyl may be obtained by reacting an aminonaphthol sulphonic acid of formula (4) with an isocyanate of the formula $R^1.NCO$ where $R^1$ is optionally substituted alkyl or optionally substituted cycloalkyl.

This reaction may be carried out in aqueous medium at essentially neutral pH and ambient temperatures. There may be some concomitant formation of a urethane by reaction of the OH group in the aminonaphthol sulphonic acid in addition to reaction at the amino group but this unwanted urethane group may be removed by hydrolysis e.g. by increasing the pH of the reaction mixture for a short time.

It will be appreciated that this method is applicable only to products of formula (1) in which R is H and in which $R^1$ does not bear isocyanate reactive substituents such as OH.

As examples of isocyanates $R^1.NCO$ for use in this process there may be mentioned methyl isocyanate, propyl isocyanate and hexyl isocyanate.

As with the previous methods of manufacturing the naphthol sulphonic acids of formula (1) the product may be isolated from the reaction mixture by conventional methods or used with isolation in further reactions such as coupling.

The naphthol sulphonic acids of formula (1) are valuable chemical intermediates especially for use as coupling components in the manufacture of azo dyestuffs. Thus a further feature of the present invention is azo dyestuffs of the formula:

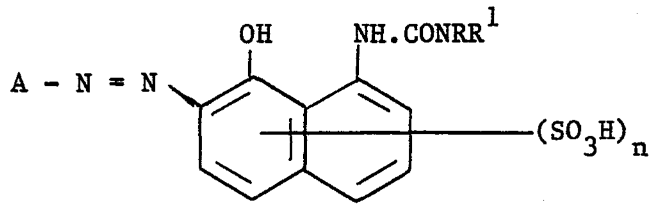

(6)

where A or its precursor is the radical of a diazotisable amine and n, R and $R^1$ have the meanings given above.

In many instances the radical A will correspond directly with the radical of the diazotisable amine used to prepare the dyestuff. This preparation will be by conventional means i.e. the amine $A.NH_2$ will be diazotised e.g. by reaction with sodium nitrite/acid at 0°C and the resulting diazonium salt solution added to a solution of the naphthol sulphonic acid of formula (1) at 0 to 30°C.

The amine $A.NH_2$ may itself contain one or more azo groups leading to the formation of a dis or polyazo dye of formula (6).

As specific examples of amines $A.NH_2$ there may be mentioned

aniline-2,3- and 4-sulphonic acids
aniline-2,5-disulphonic acid
aniline-3,5-disulphonic acid
aniline-2,4-disulphonic acid
4- and 5-sulphoanthranilic acids
2-nitroaniline-4-sulphonic acid
4-nitroaniline-2-sulphonic acid
3-nitroaniline-6-sulphonic acid
5-nitro-2-aminoanisole-4-sulphonic acid
2-methylaniline-4,5-disulphonic acid
2,5-dichloroaniline-4-sulphonic acid
4-methylaniline-2-sulphonic acid
5-chloro-4-methylaniline-2-sulphonic acid
4-methoxyaniline-2-sulphonic acid
4-chloroaniline-3-sulphonic acid
5-acetylaminoaniline-2,4-disulphonic acid
anthranilic acid

2-aminoterephthalic acid
*m*-aminobenzoic acid
3- or 4-aminophthalic acids
*p*-aminobenzoic acid
5-nitroanthranilic acid
6-nitro-3-aminobenzoic acid
5-nitro-2-aminoterephthalic acid
1-naphthylamine-4-sulphonic acid
2-naphthylamine-4,8-disulphonic acid
6-nitro-2-naphthylamine-4,8-disulphonic acid
2-naphthylamine-6,8-disulphonic acid
2-naphthylamine-5,7-disulphonic acid
3- and 4-acetylaminoaniline-6-sulphonic acids
2-aminoanisole-4- or 5-sulphonic acids
5-amino-2-hydroxybenzoic acid
1-naphthylamine-5-, 6- or 7-sulphonic acids
2-naphthylamine-1-, 5-, 6-, 7- or 8-sulphonic acids
1-naphthylamine-3,6-disulphonic acid
2-naphthylamine-1,5-disulphonic acid
2-naphthylamine-1,6-disulphonic acid
2-naphthylamine-6,8-disulphonic acid
6-acetylamino-2-naphthylamine-4,8-disulphonic acid
2-naphthylamine-3,6,8-trisulphonic acid
2-naphthylamine-4,6,8-trisulphonic acid
aniline
*o*-, *m*- and *p*-toluidine
2,4- and 2,5-dimethylaniline
5-methyl-2-methoxyaniline
*o*-, *m*- and *p*-chloroaniline
*o*-, *m*- and *p*-bromoaniline
and amino monoazo compounds obtained by coupling the diazonium salts of any amine listed above with any of the following:
aniline
*o*- and *m*-toluidine

o- and m-anisidine
anthranilic acid
cresidine
2,5-xylidine
2,5-dimethoxyaniline
m-aminoacetanilide
m-aminophenylurea
4-methyl-2-aminobenzoic acid
3-acetylamino-6-methylaniline
3-acetylamino-6-methoxyaniline
3-benzoylaminoaniline
1-naphthylamine-6- and 7-sulphonic acids
2-ethoxy-1-naphthylamine-6-sulphonic acid.

Dyes from such amines may provide valuable acid dyes for the colouration of substrates such as leather, wool or synthetic polyamides.

In other instances the dyestuff of formula (6) will be formed by subjecting a dyestuff of the formula:

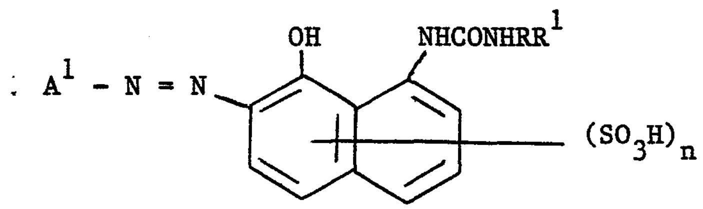

(7)

to a reaction or series of reactions which converts the radical $A^1$ to A where A, R, $R^1$ and n have the meanings given above and $A^1$ is the radical of a diazotisable amine $A^1NH_2$.

The dyestuffs of formula (7) may be obtained by conventional procedures of diazotising amine $A^1NH_2$ and coupling with a naphthol sulphonic acid of formula (1).

The reactions used to convert the radical $A^1$ to A may be of various types well known in the dyestuffs art. For example acylamino groups may be hydrolysed to form free amino groups. Conversely, amino groups may be acylated with a variety of agents such as acid chlorides or anhydrides or alkylated with alkyl halides or sulphates.

A valuable class of dyes of formula (6) are those in which A is a group of the formula:

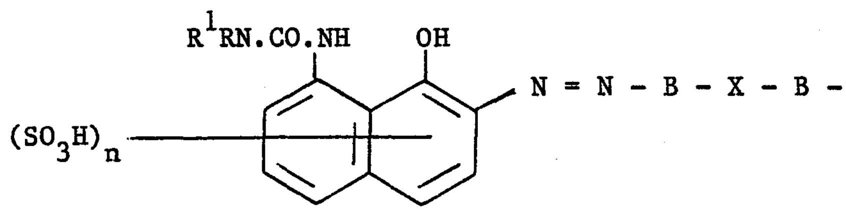

(8)

where R, $R^1$ and n have the meanings given above, B is derived from the radical of a diazotisable amine which has a substituent capable of reacting with a reagent with two reactive atoms or groups to form the bridging atom or group X. Such dyes are formed by reacting two moles of a dyestuff of formula (7) in which $A^1$ provides the precursor to B with one mole of a direactive reagent leading to the formation of the atom or group X. For example two moles of a dyestuff of formula (7) in which $A^1$ has an $NH_2$ substituent may be reacted with one mole of phosgene leading to dyes in which the entity B - X - B in formula (8) is NH.CO.NH. Alternatively the chlorides of dibasic acids $R^2(COOH)_2$ may be used in place of phosgene to give a B - X - B entity of the form $NH.CO.R^2.CO.NH$. "Doubled-up" dyes of this type can be of value as direct dyes for substrates such as paper or cotton. Direct dyes may also be obtained by linking two moles of a chlorotriazinyl dye as described below with one mole of a diamine.

A preferred class of dyestuffs of formula (6) are those in which the radical A contains a cellulose-reactive group $Q^2$ and it is especially preferred that A is of the formula:

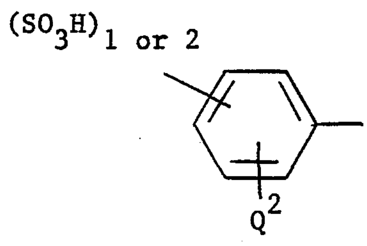

The cellulose reactive group $Q^2$ may be of any conventional type which forms a covalent bond with cellulose in the presence of an acid binding agent usually an alkali. As examples of such cellulose-reactive groups there may be mentioned vinyl sulphonyl and beta-sulphatoethylsulphonyl groups attached to a carbon atom of the dyestuff but it is usually preferred that it is of the form $NR^7Q$ where $R^7$ is lower alkyl especially H and Q is a cellulose-reactive group.

The group Q may be introduced by reacting a dyestuff of formula (7) in which the radical $A^1$ contains a $NHR^7$ group with carbyl sulphate or a compound Qhal where hal is halogen and Q is a cellulose-reactive group other than beta-sulphatoethylsulphonyl.

Alternatively a diazotisable amine containing a $NHR^7$ group may be reacted with carbyl sulphate or Qhal before it is diazotised and coupled to form a dyestuff of formula (6).

The reaction of carbyl sulphate or Qhal with the group $NHR^7$ may be achieved under conventional conditions, e.g. in an aqueous medium at a temperature of 0-100°C depending on the reactivity of the reactants. It is usually desirable to maintain the pH at say 4-8 especially 6-7 by addition of acid binding agent. The reactive group Q on the dyestuff may be subject to modifying reactions, e.g. chlorotriazinyl groups may be quaternised, in a manner well known to the reactive dye art.

The cellulose-reactive group Q is any group capable of forming a covalent bond with a cellulose substrate under alkaline conditions. The group Q may be any conventional cellulose-reactive group of this type such as those disclosed in the patent specifications and other literature on reactive dyes, e.g. UK Patent Specification No.1440948.

As examples of cellulose-reactive groups, there may be mentioned aliphatic sulphonyl groups which contain a sulphate ester

group in beta-position to the sulphur atom, e.g. beta-sulphatoethylsulphonyl groups, alpha,beta-unsaturated acyl radicals of aliphatic carboxylic acids, for example acrylic acid, alpha-chloroacrylic acid, alpha-bromoacrylic acid, propionic acid, maleic acid and mono- and dichloro maleic acids; also the acyl radicals of acids which contain a substituent which reacts with cellulose in the presence of an alkali, e.g. the radical of a halogenated aliphatic acid such as chloroacetic acid, beta-chloro- and beta-bromopropionic acids and alpha,beta-dichloro- and dibromopropionic acids or radicals of vinylsulphonyl- or beta-chloroethylsulphonyl- or beta-sulphatoethylsulphonyl-endo-methylene cyclohexane carboxylic acids. Other examples of cellulose-reactive groups are

tetrafluoro*cyclo*butyl carbonyl
trifluoro*cyclo*butenyl carbonyl
tetrafluoro*cyclo*butylethenyl carbonyl
trifluoro*cyclo*butenylethenyl carbonyl
activated halogenated 1,3-dicyanobenzene radicals such as
2,4-dicyano-3,5-difluoro-6-chlorophenyl
2,4-dicyano-3,5-difluoro-6-nitrophenyl
2,4-dicyano-3,5,6-trifluorophenyl
2,4-dicyano-3,5,6-trichlorophenyl
2,4,6-tricyano-3,5-difluorophenyl
2,4,6-tricyano-3,5-dichlorophenyl

and heterocyclic radicals which contain 1, 2 or 3 nitrogen atoms in the heterocyclic ring and at least one cellulose-reactive substituent on a carbon atom of the ring.

It may be noted that many reactive groups may be defined as both heterocyclic or acyl groups since they consist of an acyl group carrying a heterocyclic substituent. For convenience in such cases where the heterocyclic ring carries the cellulose substituent these are usually referred to as heterocyclic reactive groups in this specification.

As examples of such heterocyclic radicals there may be mentioned for example:

2,3-dichloroquinoxaline-5- or 6-sulphonyl
2,3-dichloroquinoxaline-5- or 6-carbonyl
2,4-dichloroquinazolin-6- or 7-sulphonyl
2,4,6-trichloroquinazolin-7- or 8-sulphonyl
2,4,7- or 2,4,8-trichloroquinazolin-6-sulphonyl
2,4-dichloroquinazolin-6-carbonyl
1,4-dichlorophthalazine-6-carbonyl
4,5-dichloropyridazon-1-yl-ethylcarbonyl
2,4-dichloropyrimidine-5-carbonyl
4-(4',5'-dichloropyridaz-6'-on-1-yl)benzoyl
2-chlorobenzthiazole-6-carbonyl
3,6-dichloropyrazin-4-carbonyl
4-(4',5'-dichloropyridaz-6'-on-1'-yl)phenylsulphonyl
activated 4,6-dihalopyridin-2-yl and 2,6-dihalopyridin-4-yl groups such as
3,4,5,6-tetrafluoropyridin-2-yl
2,3,5,6-tetrafluoropyridin-4-yl
2,4,6-trifluoro-3-cyanopyridin-4-yl
2,5,6-trichloro-3-cyanopyridin-4-yl
2,6-difluoro-3-cyano-5-chloropyridin-4-yl
2,6-difluoro-3,5-dichloropyridin-4-yl
and more particularly triazinyl or pyrimidinyl groups.

Examples of particular pyrimidinyl groups are pyrimidin-2-yl or -4-yl groups having a cellulose-reactive atom or group especially Cl, Br or F in at least one of the remaining 2, 4 and 6 positions. The 5-position may carry various substituents such as Cl or CN which are not normally cellulose-reactive in themselves but enhance the reactivity of substituents in other positions of the pyrimidine ring. As specific examples of such pyrimidinyl groups there may be mentioned:

2,6-dichloropyrimidin-4-yl
4,6-dichloropyrimidin-2-yl
2,5,6-trichloropyrimidin-4-yl
4,5,6-trichloropyrimidin-2-yl
5-chloro-2-methylsulphonyl-6-methylpyrimidin-4-yl

2,6-dichloro-5-cyanopyrimidin-4-yl
4,6-dichloro-5-cyanopyrimidin-2-yl
2,6-difluoro-5-chloropyrimidin-4-yl
4,6-difluoro-5-chloropyrimidin-2-yl
2,6-difluoro-5-cyanopyrimidin-4-yl
4,6-difluoro-5-cyanopyrimidin-2-yl.

Examples of particular triazinyl groups are triazin-2-yl groups having cellulose-reactive atoms or groups on one or both of the 4 and 6 positions. In this instance a wide range of cellulose-reactive atoms or groups are available such as activated aryloxy or various groups linked through a sulphur atom but the preferred reactive atoms or groups are F, Br or especially Cl, quaternary ammonium groups such as tri-lower alkyl ammonium, e.g. $(CH_3)_3N^+$- and pyridinium groups especially those derived from pyridine carboxylic acids in particular from nicotinic acid.

The triazinyl groups having only one reactive atom or group on the nucleus in the 4- or 6-position may have a substituent not reactive to cellulose in the remaining 4- or 6-position.

As examples of such non-reactive substituents there may be mentioned alkyl or aryl thio groups, alkoxy or aryloxy groups and optionally substituted amino groups.

Preferred forms of these groups include lower, i.e. $C_{1-4}$ alkoxy, e.g. methoxy, ethoxy, n-propoxy and iso-propoxy, butoxy and lower alkoxy lower alkoxy, e.g. beta-methoxyethoxy, beta-ethoxyethoxy; phenoxy and sulphophenoxy; amino; lower alkylamino, e.g. methylamino, ethylamino, butylamino, di(lower alkyl)amino, e.g. dimethylamino, diethylamino, methylethylamino, dibutylamino and groups of the latter two types in which the alkyl groups are substituted in particular by OH, CN or $SO_3H$, e.g. beta-hydroxyethylamino, di(beta-hydroxyethyl) amino, beta-cyanoethylamino, di(beta-cyanoethyl)amino, beta-sulpho- ethylamino, beta-hydroxypropylamino, (beta-hydroxybutyl)-ethylamino and (beta-hydroxyethyl)methylamino; cycloalkylamino, e.g. cyclohexylamino; cyclic amino, e.g. morpholino, piperazino or piperidine, naphthylamino substituted by 1, 2 or 3 $SO_3H$ groups and optionally substituted phenylamino groups.

As a particularly preferred form of the optionally substituted phenylamino groups there may be mentioned groups of the formula:

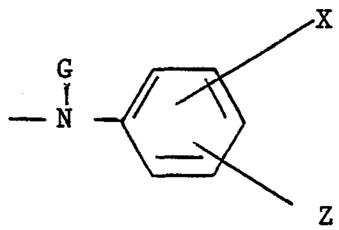

where G is H, methyl, ethyl, omega-sulphomethyl, beta-carboxy-, beta-hydroxy- or beta-cyanoethyl and Z and X are each independently selected from H, COOH, $SO_3H$, $CH_3$, $C_2H_5$, $OCH_3$, $OC_2H_5$, Cl, Br, CN, $NO_2$ and $NHCOCH_3$.

As specific examples of such groups there may be mentioned:

anilino

o-, m- and p-sulphoanilino

o-, m- and p-carboxyanilino

4- and 5-sulpho-2-carboxyanilino

4- and 5-sulpho-o-tolylamino

2,4-, 2,5- and 3,5-disulphoanilino

2,4-dicarboxyanilino

4- and 5-sulpho-2-methoxyanilino

N-methyl-o-, m- and p-sulphoanilino

N-omega-sulphomethylanilino

N-(beta-hydroxyethyl)-3-sulphoanilino.

If desired, the cellulose-reactive group Q may take the form:

$$- Ht - Dm - Q^1$$

where Ht is a s-triazine nucleus which contains a cellulose-reactive atom or group, Dm represents a diamine residue linking Ht and $Q^1$ by the two amino groups and $Q^1$ is a cellulose-reactive group having the meaning stated above for Q.

In particular it is usually preferred that the cellulose-reactive atom or group on Ht is F, Br or especially Cl and that $Q^1$ is a s-triazine residue as defined above for Q.

When symbol Q represents a s-triazine ring containing a halogen atom and a non-reactive substituent, the latter can be the residue of a coloured amine, e.g. of the azo, anthraquinone or phthalocyanine series, but more especially a residue of the dyestuff of formula (7) so that the dyestuff as a whole contains two residues of these dyes bearing $NR^7$-groups linked by a halo-s-triazine radical or two such residues linked through two halo-s-triazine groups and a diamine radical Dm as given above. In these dyestuffs the halogen may be F or Br but it is usually preferred that it is Cl.

As examples of the diamines $DmH_2$ which may provide the residue Dm there may be mentioned:

heterocyclic diamines e.g. piperazine

aliphatic diamines e.g. alkylene, hydroxyalkylene or sulphato alkylene diamines especially those having 2-6 carbon atoms e.g.

ethylene diamine

1,2- and 1,3-propylene diamines

1,6-diaminohexane

beta-hydroxyethylaminoethylamine

2-hydroxy-1,3-diaminopropane

2-sulphato-1,3-diaminopropane

other alpha, omega-diamino aliphatic compounds e.g.

triethylene tetramine

di-(beta-aminoethyl)ether

naphthylene diamine sulphonic acids e.g.
2,6-diaminonaphthalene-1,5- and 4,8-disulphonic acids
1,5-diaminonaphthalene-3,7-disulphonic acids
and diamine derivatives of mono- and di-cyclic compounds of the benzene series, e.g. phenylene diamines and their mono- and disulphonic acids, e.g.
m- and p-phenylene diamines
1,3-phenylenediamine-5-sulphonic and 4,6-disulphonic acids
1,4-phenylenediamine-2-sulphonic and 2,5-disulphonic acids
diamines of the formula:

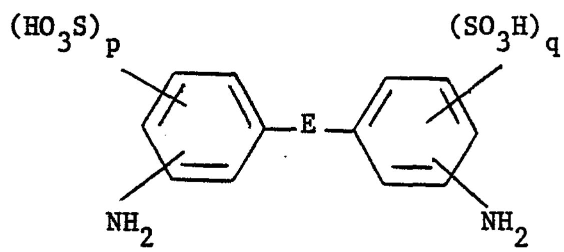

where p and q are each 0 or 1 and E is a direct link or a group selected from $SO_2$, $CH_2$, $C_2H_4$, NH, NHCONH, e.g.
4,4'-diaminostilbene-2,2'-disulphonic acid
benzidine-2-sulphonic and 2,2'-disulphonic acids
3,3'- and 4,4'-diaminodiphenylureas
4,4'-diaminodiphenylurea-2,2'- and 3,3'-disulphonic acids
4,4'-diaminodiphenylamine-2,2'-disulphonic acid
4,4'-diaminodiphenylmethane
4,4'-diaminodiphenylmethane-2,2'-disulphonic acid
4,4'-diaminophenyl sulphone
and the N-lower alkyl and N,N'-di(lower alkyl) derivatives of the above diamines, e.g.
4-methylamino and 4-ethylaminoaniline-2-sulphonic acids
N,N'-dimethyl-4,4'-diaminodiphenylamino-2,2'-disulphonic acid.

Cellulose-reactive dyes of formula (6) usually exhibit a good balance of water solubility and colouring power often superior to

closely related dyes such as those from acetyl- or benzoyl amino naphthol sulphonic acid coupling components. Further these dyes show good fastness to light and washing and are especially valuable in discharge printing applications.

The invention is illustrated by the following Examples.

Example 1

H-acid (0.05M) as the monosodium salt was added to 100ml water and dissolved by the addition of sufficient 2M sodium hydroxide solution to raise the pH to 7.0. Sodium cyanate (5g) was added followed by 2M hydrochloric acid (30ml) added dropwise over 15 minutes. The reaction mixture was stirred at 20°C and the course of the reaction was followed by HPLC. After 4 hours no H-acid could be detected either by HPLC or by reaction with Erlichs reagent which gives a strong orange colouration with H-acid.

The solution was used to prepare azo dyestuffs directly.

The structure of the product is believed to be formula (3) with $R = R^1 = H$.

Example 2

H-acid (0.1M) was stirred into water (250ml) and the pH raised to 5.8 by addition of sodium bicarbonate. N-propyl isocyanate (20ml) was added dropwise to the solution over 1 hour and, after stirring for a further 30 minutes, the mixture was analysed by HPLC. No H-acid was detected in the reaction but two products were observed. Sodium carbonate was then added to raise the pH to 9.5 and the mixture stirred a further 1 hour. HPLC analysis now showed only one product to be present. (The second product was assumed to be the urethane prepared by reaction of the isocyanate at both O and N atoms and as expected the urethane was hydrolysed at pH 9.5).

The reaction mixture was adjusted to pH 7 by addition of hydrochloric acid and then salted to 10%. The crystalline precipitate was collected by filtration and dried at 30°C. Yield of grey powder = 39.6g.

The structure of the product is believed to be formula (3) in which R = H and $R^1$ = n-propyl.

Examples 3 - 9

Cyclic urethane of formula (5) with 3,6-sulphonic acid groups (M/10) was stirred in methanol (200ml) and ethanolamine (50ml) was added. The mixture was refluxed for 20 hours.

The product in solution is believed to have the structure given by formula (3) with R = H and $R^1$ = $CH_2CH_2OH$. The methanolic solution was used directly to prepare dyestuffs.

By replacing the ethanolamine with an equivalent amount of the amines detailed below solutions were prepared of other naphthol sulphonic acids of formula (3) with R and $R^1$ as given.

| Example | Amine | R | $R^1$ |
|---|---|---|---|
| 4 | methylamine | H | $CH_3$ |
| 5 | dimethylamine | $CH_3$ | $CH_3$ |
| 6 | ethylamine | H | $C_2H_5$ |
| 7 | n-propylamine | H | n-$C_3H_7$ |
| 8 | gamma-dimethylaminopropylamine | H | $(CH_2)_3.N(CH_3)_2$ |
| 9 | n-butylamine | H | n-$C_4H_9$ |

The cyclic urethane for use in the above was obtained by either of the following methods.

A. The procedure of Example 1 was repeated. When the reaction was deemed complete i.e. when no H-acid could be detected in the reaction mixture the mixture was heated to 65°C for 2 hours. Completion of cyclisation was shown by the absence of a colour reaction when a sample of the solution was contacted with diazotised p-chloraniline solution. The solution was cooled to 20°C and salted to 20%. The precipitated product was collected by filtration, washed with 20% brine and dried at 60°C.

Yield of grey solid = 20g.

B. Method of Balaban King, J.C.S. 1927, 3091. Phosgene (0.32M) was bubbled through a neutral solution of H-acid (0.3M) in water (1250ml) at ambient temperature. The pH of the solution was allowed to fall to 5.0 and was maintained at 5.0 by addition of sodium hydroxide solution. After 3.5 hours analysis by HPLC showed the reaction to be complete. The pH was then adjusted to 2.0 and the product collected by filtration, washed with brine and dried. Elementary analysis gave C 28.5%, H 2.4%, N 3.0%, S 13.1%. The sample contained 17.4% water. When adjusted accordingly the required analytical values are: C 28.65%, H 2.8%, N 3.05%, S 13.9%.

The product is identical (HPLC and IR spectrum) with that obtained by method A.

The yield of pale grey powder was 136g.

Example 10

A solution of m-phenylenediamine sulphonic acid (M/40) in water (50ml) and 2M sodium hydroxide (12.5ml) was added to an ice cold suspension of cyanuric chloride (4.63g) in water (200ml). After stirring 1 hour the pH was raised to 7.0 by addition of 2M sodium hydroxide and 2M sodium nitrite (12.5ml) was added followed by 2M hydrochloric acid (32ml). The pale yellow suspension was stirred at <5°C for 1 hour.

A solution of the product of Example 1 containing m/40 of the ureido naphthol sulphonic acid was diluted to 200 ml and the pH was adjusted to 3-4 by addition of hydrochloric acid. The diazonium salt suspension was then added and the pH slowly raised to 5.5 by addition of sodium hydroxide solution. When the coupling reaction was judged to be complete the resulting dyestuff was precipitated from solution by salting to 10%, collected by filtration and dried.

The dyestuff has the structure:

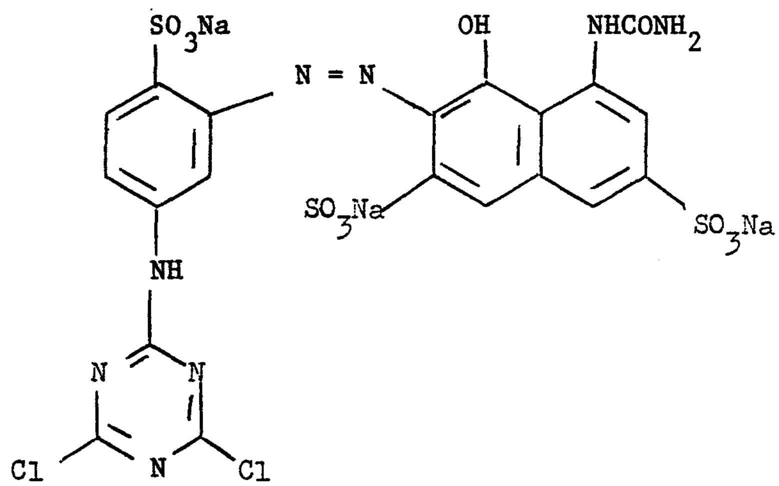

It has high solubility in cold water and dyes cotton under both pad-batch and exhaust dyeing conditions to give fully dischargeable bright red shades. It shows exceptionally good build-up properties, particularly by pad-batch application.

Examples 11 - 14

By repeating the procedure of Example 10 replacing the product of Example 1 with equivalent amounts of the products of Examples 2, 3, 5 and 8 respectively, similar dyes were obtained.

Examples 15 - 19

The dye preparations given in Examples 10 - 14 were repeated replacing the cyanuric chloride with an equivalent amount of 5-cyano-2,3,6-trichloropyrimidine to yield similar dyes.

The dichlorotriazinyl dyes obtained in Examples 10 - 14 inclusive were used to prepare monochlorotriazinyl products by the following general methods.

Example 20

The dyestuff of Example 10 was estimated by titration with 0.1N titanous chloride solution.

232g (at Mol.In.3781g) were dissolved in 1600ml cold water with stirring and a solution of metanilic acid, (10.66g) dissolved in water

(100ml) and sufficient 2N sodium carbonate solution to raise the pH to 6-7 by further small additions of sodium carbonate solution. When the reaction was judged (by HPLC) to be complete the mixture was cooled whereupon the product crystallised from solution. It was collected by filtration and dried. The product had the structure:

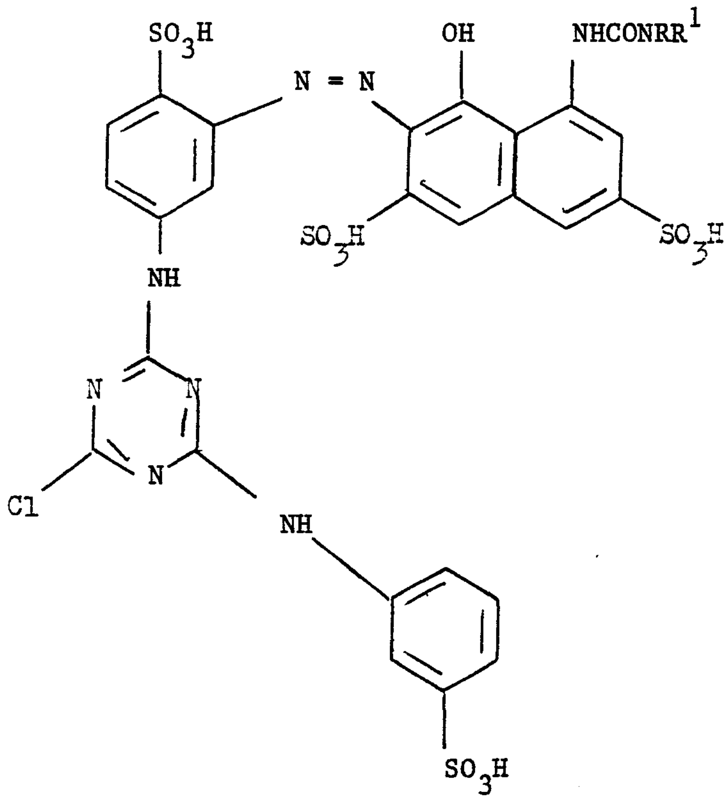

where R = $R^1$ = H.

It is applied to cellulosic textile materials under alkaline conditions to give bright red shades having very high fastness to wet treatments and to light.

By similar methods, the dyestuffs of Examples 11 - 14 were converted to products in which

Example 21 R = H; $R^1 = -CH_2CH_2CH_3$

Example 22 R = H; $R^1 = -C_2H_4OH$

Example 23 $R = -CH_3$; $R^1 = -CH_3$

Example 24 R = H; $R^1 = -C_3H_6N(CH_3)_2$

Example 25

A further 68.8g sample of the dyestuff of Example 10 (at Mol.In.1089g) were dissolved in 800ml of cold water. A solution of p-phenylenediamine (3.4g) dissolved in water (100ml) by the addition of 2N hydrochloric acid, was added and the pH of the mixture was adjusted to pH 6-7. The temperature was raised to 30°C until the reaction was judged by HPLC to be complete and then allowed to fall to ambient temperature. The product which had crystallised was collected by filtration and dried.

It had the structure:

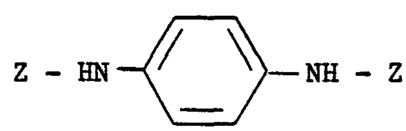

wherein Z is a monoazo structure of the formula:

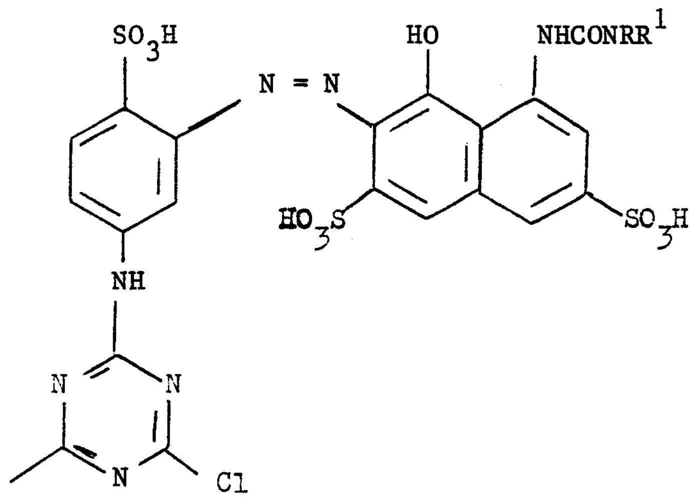

and $R = R^1 = H$.

The dye can be applied to cellulosic textile materials under typical alkaline hot-dyeing conditions.

Similarly the dyestuffs of Examples 11 - 14 were converted to dyestuffs of the formula;

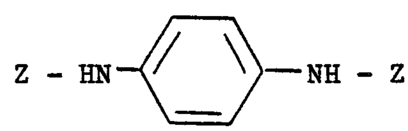

wherein:

| | | |
|---|---|---|
| Example 26 | $R = H$; | $R^1 = -CH_2CH_2CH_3$ |
| Example 27 | $R = H$; | $R^1 = -C_2H_4OH$ |
| Example 28 | $R = -CH_3$; | $R^1 = -CH_3$ |
| Example 29 | $R = H$; | $R^1 = -C_3H_6N(CH_3)_2$ |

## Examples 30 - 32

Dyestuffs suitable for application by typical direct dyeing processes may be prepared as in Examples 25 - 29 above by reaction with amines.

Thus the dyestuff of Example 25 where $R = R^1 = H$ was treated with excess monoethanolamine in aqueous solution at 80°C to yield a product with structure:

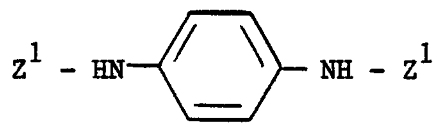

wherein $Z^1$ is a monoazo structure of the formula:

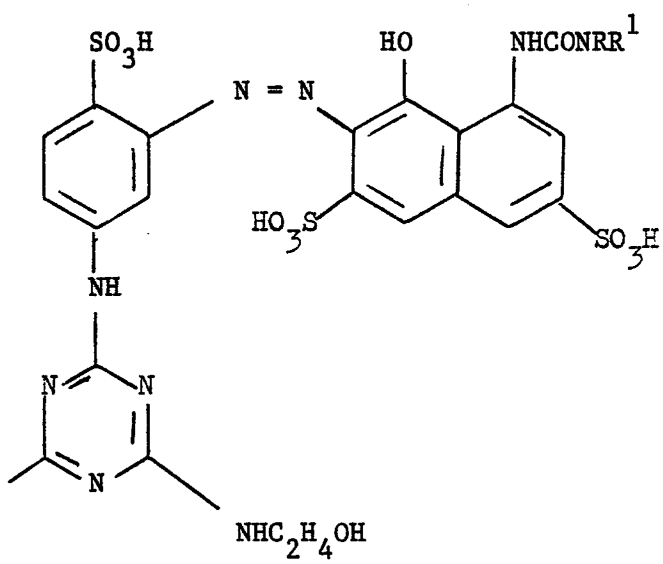

and $R = R^1 = H$.

The product was applied to cellulosic textile material under direct dyeing conditions, i.e. applied from a boiling aqueous dyebath in the presence of sodium chloride or sodium sulphate, to yield a full bright red shade having good fastness to light and to wet treatments.

By replacing the ethanolamine with diethanolamine and with morpholine, dyes were produced with similarly good yield and build-up under direct dyeing conditions.

Example 33

30.3g of p-(sodium beta-sulphatoethylsulphonyl)aniline were dissolved in 250ml of water at 0-5°C and treated successively with 55ml of 2N molar sodium nitrite solution and 150ml of 2N hydrochloric acid solution.

The resultant diazonium chloride solution was added to a solution containing 0.1 mole of the coupling component prepared as described in Example 1 at 0-5°C and pH 6. When the coupling reaction was complete, the dyestuff was precipitated from solution by the addition of sodium chloride, collected by filtration and dried. The dye had high solubility in cold water and dyed cotton under pad-batch, exhaust dyeing, printing and continuous (steam or thermofix) dyeing conditions to give fully dischargeable bright red shades. It showed exceptionally good build-up properties particularly in pad-batch application. The dyestuff has the structure:

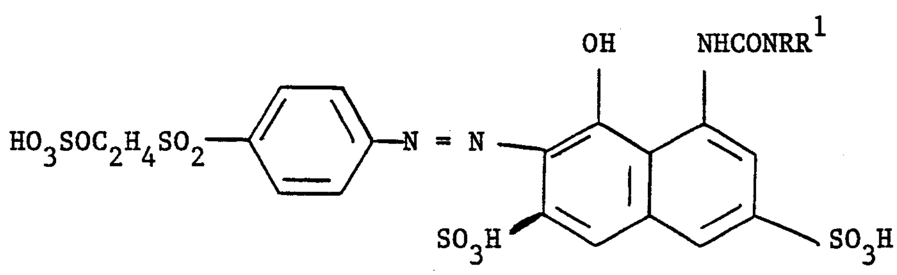

where $R = R^1 = H$.

Examples 34 - 37

By replacing the product of Example 1 in the method of Example 33 with equivalent amounts of the products of Examples 2, 3, 5 and 8 respectively, similar dyes were obtained.

Examples 38 - 43

By replacing the p-(sodium beta-sulphatoethylsulphonyl)aniline in Examples 33 - 37 with the corresponding m-isomer a second series of yellower shade bright red dyestuffs was produced.

Example 44

26.1g of p-dodecylaniline were dissolved in 100ml of glacial acetic acid and 50ml of water and treated successively with 55ml of 2 molar sodium nitrite solution and 25ml of 36% hydrochloric acid solution at 0-5°C. After stirring to complete the diazotisation reaction, the suspension was added to a solution containing 0.1 mole of the naphthol sulphonic acid of Example 3. 50g of sodium acetate were added and the mixture stirred at 0-10°C for 12 hours. The resultant dyestuff precipitate was collected and washed with cold water then dried. 53g of a dyestuff having the structure:

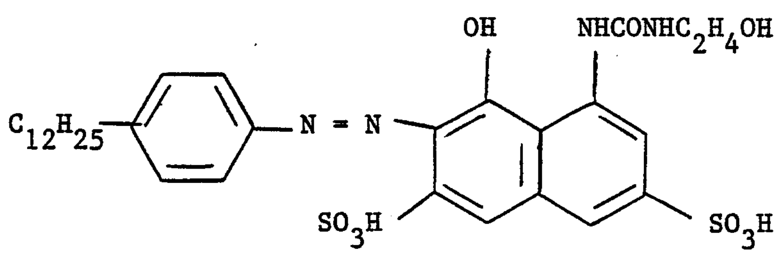

were obtained. It dyes polyamide textile materials, particularly nylon and wool from weakly acid dyebaths to give bright bluish-red shades with exceptionally high fastness to wet treatments.

CLAIMS

1. A naphthol sulphonic acid of the formula:

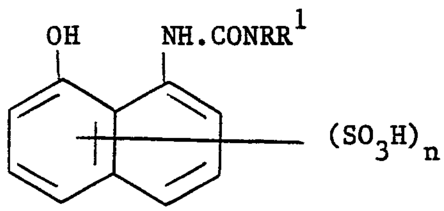

(1)

where n is 1 or 2;

R is H or an optionally substituted alkyl;

$R^1$ is H, optionally substituted alkyl or optionally substituted cycloalkyl,

or R and $R^1$ together with the nitrogen atom form a heterocyclic ring.

2. A naphthol sulphonic acid according to claim 1 having the formula:

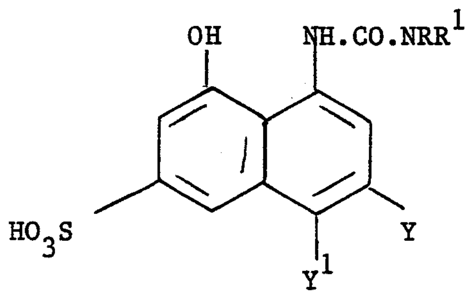

(2)

wherein R and $R^1$ have the meanings given in claim 1 and one of $Y^1$ and Y is $SO_3H$ and the other is H.

3. A naphthol sulphonic acid according to claim 2 having the formula:

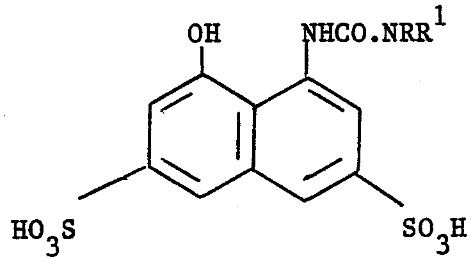

(3)

wherein each of R and $R^1$, independently is H or $C_{1-4}$ alkyl which may optionally be substituted by hydroxy, cyano, mono- or di-$C_{1-4}$ alkylamino or hydroxy $C_{1-4}$ alkoxy or R and $R^1$ together are $-(CH_2)_4-$, $-(CH_2)_5-$, $-(CH_2)_2O(CH_2)_2-$ or $-(CH_2)_2NH(CH_2)_2-$.

4. A method for the preparation of a naphthol sulphonic acid as defined in claim 1 wherein R = $R^1$ = H which comprises reacting an aminonaphthol sulphonic acid of the formula:

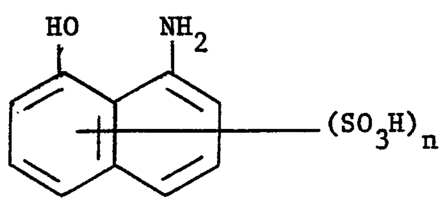

where n is 1 or 2 with a metal cyanate under acid conditions.

5. A method for the preparation of a naphthol sulphonic acid as defined in claim 1 which comprises reacting a naphthalene compound of the formula:

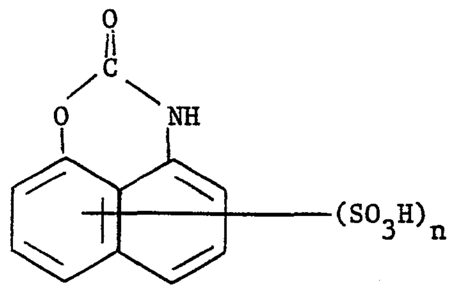

with an amine of the formula $HNRR^1$, the symbols R, $R^1$ and n having the meanings given in claim 1.

6. A method for the preparation of a naphthol sulphonic acid as defined in claim 1 wherein R is H and $R^1$ is optionally substituted alkyl or optionally substituted cycloalkyl which comprises reacting an aminonaphthol sulphonic acid of the formula:

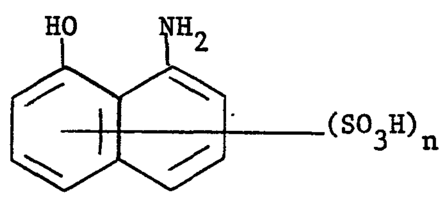

with an isocyanate of the formula $R^1NCO$.

7. A process for the production of an azo dye which comprises coupling a diazotised aromatic amine with a naphthol sulphonic acid as defined in claim 1.

KS/BH
9.7.84.

0138303

Application number

European Patent Office

# EUROPEAN SEARCH REPORT

EP 84304797.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | AT - B - 72 298 (FARBENFABRIKEN VORM. FRIEDR. BAYER & CO.)<br>* Page 1; claim 1 *<br>-- | 1,7 | C 07 C 143/66<br>C 09 B 29/30 |
| A | AT - B - 296 274 (FARBENFABRIKEN BAYER AKTIENGESELLSCHAFT)<br>* Claim 1; page 5, lines 13-15 *<br>-- | 1,7 | |
| A | EP - A1 - 0 064 693 (BAYER AG)<br>* Abstract *<br>-- | 1 | |
| A | US - A - 4 127 602 (SEYMOUR BERNSTEIN et al.)<br>* Claim 1 *<br>-- | 1 | |
| A | US - A - 4 046 805 (SEYMOUR BERNSTEIN et al.)<br>* Claim 1 *<br>-- | 1 | TECHNICAL FIELDS SEARCHED (Int. Cl.4)<br>C 07 C 143/00<br>C 07 C 127/00 |
| A | DE - A - 2 311 880 (IMPERIAL CHEMICAL INDUSTRIES LTD.)<br>* Claim 1 *<br>---- | 1,7 | |

The present search report has been drawn up for all claims

| Place of search | Date of completion of the search | Examiner |
|---|---|---|
| VIENNA | 29-10-1984 | REIF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82